# EUROPEAN PATENT SPECIFICATION

(11) **EP 2 988 775 B1**
(45) Date of publication and mention of the grant of the patent: **02.06.2021**
(21) Application number: 14788739.2
(22) Date of filing: 25.04.2014
(51) Int. Cl.: A61K 39/00, A61K 39/38, C40B 30/04, A61K 35/17, G01N 33/50, A61P 43/00

(54) **IMMUNE MODULATION FOR TREATMENT OF AUTOIMMUNE DISEASE**
IMMUNMODULATION ZUR BEHANDLUNG VON AUTOIMMUNERKRANKUNGEN
MODULATION IMMUNE POUR LE TRAITEMENT D'UNE MALADIE AUTO-IMMUNE

(30) Priority: 26.04.2013 US 201313871730
(43) Date of publication of application: 02.03.2016
(73) Proprietor: Enzo Biochem, Inc., New York, NY 10022 (US)
(72) Inventor: NUSSENBLATT, Robert, DECEASED (US); LIU, Baoying, North Potomac, MD 20878 (US); WEI, Lai, Rockville, MD 20852 (US); RABBANI, Elazar, New York, NY 10003 (US); DONEGAN, James, J., Amesbury, MA 01913 (US)
(74) Representative: Vossius & Partner Patentanwälte Rechtsanwälte mbB
(86) International application number: PCT/US2014/035549
(87) International publication number: WO 2014/176555

(56) References cited:
- EP-A1- 2 113 560
- WO-A1-2013/036914
- US-A1- 2009 010 885
- US-B1- 6 790 447
- HOWARD L. WEINER ET AL: "Oral tolerance", IMMUNOLOGICAL REVIEWS., vol. 241, no. 1, 13 May 2011 (2011-05-13), pages 241-259, XP055302913, US ISSN: 0105-2896, DOI: 10.1111/j.1600-065X.2011.01017.x
- BHARATI MATTA ET AL: "Antigen-specific tolerance inhibits autoimmune uveitis in pre-sensitized animals by deletion and CD4+CD25+ T-regulatory cells", IMMUNOLOGY AND CELL BIOLOGY, vol. 88, no. 2, 3 November 2009 (2009-11-03), pages 187-196, XP055302955, AU ISSN: 0818-9641, DOI: 10.1038/icb.2009.83
- STEPHAN R. THURAU ET AL: "Oral tolerance with an HLA-peptide mimicking retinal autoantigen as a treatment of autoimmune uveitis", IMMUNOLOGY LETTERS., vol. 68, no. 2-3, 1 June 1999 (1999-06-01) , pages 205-212, XP055303248, NL ISSN: 0165-2478, DOI: 10.1016/S0165-2478(99)00071-1
- LU ET AL.: 'Major peptide autoepitopes for nucleosome-specific T cells of human lupus.' J CLIN INVEST vol. 104, no. 3, 01 August 1999, pages 345 - 355, XP055289116 DOI: 10.1172/JCI6801

### SEQUENCE LISTING

<110> NUSSENBLATT, RICHARD LIU, BAOYING WEI, LAI RABBANI, ELAZAR DONEGAN, JAMES J.
<120> IMMUNE MODULATION FOR TREATMENT OF AUTOIMMUNE DISEASE
<130> ENZ-107(CIP)
<140> 13/871,730
   <141> 2013-04-26
<160> 2
<170> PatentIn version 3.5
<210> 1
   <211> 20
   <212> PRT
   <213> Artificial Sequence
<220>
   <223> Description of Artificial Sequence: Synthetic peptide
<400> 1
   Gly Glu Pro Ile Pro Val Thr Val Asp Val Thr Asn Asn Thr Glu Lys
   1               5                   10                  15
   Thr Val Lys Lys
               20
<210> 2
   <211> 14
   <212> PRT
   <213> Artificial Sequence
<220>
   <223> Description of Artificial Sequence: Synthetic peptide
<400> 2
   Ala Leu Asn Glu Asp Leu Ser Ser Trp Thr Ala Ala Asp Thr
   1               5                   10

## Description

### 3. BACKGROUND

The mammalian immune system has two contrasting functions that must co-exist for the health of the organism. On the one hand, the immune system recognizes foreign agents, *e*.*g*., "non-self' agents such as bacteria or viruses that it attacks and destroys to restore health to an infected organism. On the other hand, the immune system recognizes the tissues of the organism and non-pathogenic "foreign" substances that are ingested (*e.g.,* food) so that the "self' is not attacked and the organism survives. In order for the regulation of these two functions to co-exist, the immune system must constantly correctly identify "self' and "non-self' to mount a proper response and to maintain a balance between action and selective inaction with respect to various challenges.

Autoimmune diseases result from an imbalance of the immune system, which becomes unable to distinguish "self' from "non-self' and mounts an inappropriate immune response to healthy tissues of the organism. The result of this imbalance is inflammation and tissue damage, which is often irreversible. Today, an autoimmune etiology is known or suspected to play a role in numerous seemingly unrelated diseases such as, uveitis, Crohn's disease, diabetes mellitus type I, lupus erythematosus, myasthenia gravis, psoriasis and rheumatoid arthritis. Increasing evidence suggests that immune mediated mechanisms also play an important role in the pathogenesis of age-related macular degeneration ("AMD"), the leading cause of blindness in the United States and the leading cause of blindness in people over 60 years of age. *See, e.g.,* Tarallo et al. (2012) Cell 149:847-859; Rosenbaum (2012) N Engl J Med. 367(8):768-770; Nussenblatt and Ferris (2007) AMD and the Immune System 144(4):618-626; Becerril et al. (2009) Cellular & Molecular Immunology 6(4):303-307. Accordingly, although AMD has traditionally been thought of as a disease confined to the eye, recent research suggests that it is a systemic immunological disease with local expression.

Current therapies for autoimmune diseases involve suppression of the immune system to mitigate the improper attack on "self' tissues. However, immune suppressive therapies tend to be non-selective, leading to inhibition of not only the aberrant autoimmune response, and but also of healthy responses to pathogens. Accordingly, immunosuppressive therapies can leave patients susceptible to infections, cancer and drug toxicity. Furthermore, suppression of the immune system only addresses one of the two functions of the immune system, which results in further unbalancing the system.

One approach for suppressing diseases that have an autoimmune component is induction of specific immune tolerance to soluble antigens by applying the soluble antigen to mucosal surfaces. *See e.g.,* Weiner et al. (2011) Immunol. Rev. 241(1):241-59. These tolerizing epitopes are administered to a patient in order to upregulate the functions of regulatory T-cells, which are T-cells with particular phenotypes that suppress responder T-cells, cells that are responsible for attacking agents that are recognized as "non-self'. While induction of regulatory T-cells by oral administration of a soluble antigen is considered to be a promising approach to treatment of autoimmune diseases, the ability to produce significant numbers of regulatory T-cells has been limited and requires identification of additional strategies (such as identification of a better antigen and/or co-administration of an enhancer of immune tolerance) to induce adequate numbers of functional regulatory cells. *See, e.g*., Weiner at 249-50.

A more recent approach for suppressing autoimmune disease is to administer regulatory T-cells to the patient. *See, e.g.,* Marek-Trzonkowska et al. (2012) Diabetes Care 35:1817-20. In this study, regulatory autologous T-cells were expanded, but were not trained in the presence of an epitope. *Id.* at 1818. While this approach appears to have efficacy, it is not known whether it will provide long-term suppression of autoimmune disease. *Id.* at 1820. Furthermore, regulatory T-cell based therapies may be complicated by low numbers of regulatory T-cells in the body compared to other T-cells and their anergy, which means that they do not readily expand to provide enough cells for administration to a patient.

Accordingly, there is a need for improved methods and compositions that restore balance to the immune system of a patient suffering from an autoimmune disease by upregulating the regulatory function of the immune system.

### 4. SUMMARY

The present invention provides: a method of identifying a compound comprising an epitope from a synthetic peptide library or peptide collection that induces immune tolerance in a human patient suffering from an autoimmune disease, comprising
(a) exposing isolated human CD4⁺CD25⁺ cells to the compound,
(b) measuring the proliferation (Reg₁) of the human CD4⁺CD25⁺ cells in the presence of the compound,
(c) measuring the proliferation (Reg₂) of the human CD4⁺CD25⁺ cells in the absence of the compound, wherein the compound that induces Reg₁/Reg₂>1 is identified as the compound that induces immune tolerance in the patient;
(d) using the compound identified in step (c) as a reference sequence to search a library or collection of compounds for one or more compounds having homology to the reference sequence;
(e) identifying one or more compounds having homology to the reference sequence; and
(f) repeating steps (a)-(c) with one or more compounds identified in step (e).

In various aspects, the present disclosure is directed to a method of identifying a compound comprising an epitope that induces immune tolerance in a human patient suffering from an autoimmune disease comprising the step of identifying *in vitro* a compound from a library or collection of compounds that (a) elicits a response (RespH) from responder T-cells of a healthy individual; (b) elicits a response (RespP) from responder T-cells of the patient; (c) elicits a response (RegH) from regulatory T-cells of a healthy individual; and (d) elicits a response (RegP) from regulatory T-cells of the patient (RegP), wherein the compound that induces a response selected from a RespH/RespP<1, a RegH/RegP≥1, or a RespH/RespP<1 and a RegH/RegP≥1 is identified as the compound that induces immune tolerance. Accordingly, in certain aspects, a compound that induces immune tolerance is identified by a response in the presence of the compound of responder T-cells of a healthy individual that is lower than a response of responder T-cells of the patient. In other aspects, a compound that induces immune tolerance is identified by a response in the presence of the compound of regulatory T-cells of a healthy individual that is greater than or equal to a response of regulatory T-cells of the patient. In still other aspects, a compound that induces immune tolerance is identified by (i) a response in the presence of the compound of responder T-cells of a healthy individual that is lower than a response of responder T-cells of the patient; and (ii) a response in the presence of the compound of regulatory T-cells of a healthy individual that is greater than or equal to a response of regulatory T-cells of the patient.

In certain aspects, the disclosure relates to a method of identifying a compound comprising an epitope that induces immune tolerance in a human patient suffering from an autoimmune disease comprising the steps of (a) identifying *in vitro* a compound from a library or collection of compounds that (i) elicits a response (RespP₁) from responder T-cells of the patient; and (ii) elicits a response (RespH) from responder T-cells of a healthy individual wherein RespP₁/RespH >1 and (b) elicits a response (RespP₂) from responder T-cells of the patient in the presence of a responder T-cell antigen and regulatory T-cells, wherein RespP₂/RespP₁< 1, wherein the compound that induces a RespP₁/RespH >1 and RespP₂/RespP₁< 1 is identified as the compound that induces an immune tolerance. Accordingly, in certain aspects, a compound that induces immune tolerance in a patient is identified by (i) a response in the presence of the compound of responder T-cells of the patient that is greater than a response from responder T-cells of the patient; and (ii) a response in the presence of the compound of responder T-cells of the patient in the presence of a responder T-cell antigen and regulatory T-cells that is lower than a response from responder T-cells of the patient in the absence of a responder T-cell antigen and regulatory T-cells.

In a more specific aspect, the disclosure relates to a method of identifying a compound comprising an epitope from a library or collection of compounds that induces immune tolerance in a human patient suffering from an autoimmune disease comprising the steps of (a) exposing human CD4⁺CD25⁺ cells to a compound from a library or collection of compounds; and (b) measuring the proliferation of said CD4⁺CD25⁺ cells in the presence of the compound (R₁); and (c) measuring the proliferation of said CD4⁺CD25⁺ cells in the absence of the compound (R₂), wherein the compound that induces R₁/R₂>1 is identified as the compound that induces immune tolerance. Accordingly, in some aspects, the compound that induces immune tolerance in a patient is identified by a proliferation response of CD4⁺CD25⁺ cells in the presence of the compound that is greater than a proliferation response in the absence of the compound. In certain aspects, this assay is performed in the presence of an additional factor, such as IL-2.

In other aspects, the present disclosure relates to methods of identifying a compound comprising an epitope that induces immune tolerance in a patient suffering from an autoimmune disease using a mixed-cell assay. Thus, in some aspects, a compound comprising an epitope that induces immune tolerance in a patient suffering from an autoimmune disease is identified *in vitro* as the compound that elicits a response (RespP) from responder T-cells from the patient in the presence of regulatory T-cells from the patient that is greater than the response (RegP) elicited from the regulatory T-cells of the patient. Accordingly, in some aspects, an identified compound induces the response RespP/RegP>1.

In another aspect of a mixed-cell assay, a compound comprising an epitope that induces immune tolerance in a patient suffering from an autoimmune disease is identified *in vitro* as a compound that elicits a response (RegH) from regulatory T-cells from a healthy individual in the presence of responder T-cells from the patient that is greater than the response (RespP) elicited from the responder T-cells of the patient. Accordingly, in some aspects, an identified compound induces the response RegH>RespP.

In yet another aspect, a compound comprising an epitope that induces immune tolerance in a patient suffering from an autoimmune disease is identified *in vitro* as a compound that elicits a response (RespH) from responder T-cells from a healthy individual in the presence of regulatory T-cells from the patient that is greater than the response (RegP) elicited from the regulatory T-cells from the patient. Thus, in various aspects, an identified compound induces the response RespH>RegP.

In other aspects, a compound comprising an epitope that induces immune tolerance in a patient suffering from an autoimmune disease is identified *in vitro* as a compound that elicits a response (RespH) from responder T-cells from a healthy individual in the presence of regulatory T-cells from a healthy individual that is lower than the response (RegH) elicited from the regulatory T-cells from the healthy individual. In some aspects, an identified compound induces the response RespH<RegH.

In still other aspects, the present disclosure relates to a method of identifying a compound comprising an epitope that induces immune tolerance in a patient suffering from an autoimmune disease comprising the step of identifying *in vitro* a compound from a library or collection of compounds that (a) elicits a response (RespH) from responder T-cells of a healthy individual and (b) elicits a response (RespP) from responder T-cells of the patient, wherein the compound that induces a RespH/RespP<1 is identified as the compound that induces immune tolerance. Accordingly, in some aspects, the compound induces a response from responder T-cells of the patient that is greater than the response elicited from responder T-cells of a healthy individual.

It should be noted that the indefinite articles "a" and "an" and the definite article "the" are used in the present application to mean one or more unless the context clearly dictates otherwise. Further, the term "or" is used in the present application to mean the disjunctive "or" or the conjunctive "and."

Any discussion of documents, acts, materials, devices, articles or the like in this specification is solely for the purpose of providing a context for the present disclosure. It is not to be taken as an admission that any or all of these matters form part of the prior art or were common general knowledge in the field relevant to the present disclosure as it existed anywhere before the priority date of this application.

The features and advantages of the disclosure will become further apparent from the following detailed description.

### 5. BRIEF DESCRIPTION OF THE FIGURES

FIG. 1 provides the results of lymphocyte proliferation studies in AMD patients and normal controls in response stimulation with human S-antigen peptides.
FIG. 2 provides the results of lymphocyte proliferation studies in AMD patients having small, intermediate and large drusen and normal controls in response to stimulation with human S-antigen peptide 23 ("P-23").

### 6. DETAILED DESCRIPTION

In certain aspects, methods are presented for identifying a compound comprising an epitope that induces immune tolerance in a patient suffering from an autoimmune disease. In certain embodiments as defined in the claims, the compounds are identified from a synthetic peptide library or peptide collection.

As used herein, the term "patient" refers to humans and non-human animals. In some aspects, the patient suffers from an autoimmune disease due to one or more factors described herein. In certain aspects, the patient suffers from an autoimmune disease due to the presence of dysfunctional regulatory T-cells. As used herein, the term "dysfunctional" when referring to regulatory T-cells means that regulatory T-cell function in the patient is at least about 5%, at least about 10%, at least about 20%, at least about 30% or more lower than regulatory T-cell function in a healthy individual when comparing the same number of cells from the patient ant the healthy individual. In other aspects, the patient suffers from an autoimmune disease due to the presence of lower numbers of regulatory T-cells as compared to numbers of regulatory T-cells in a healthy individual. In these aspects, the patient has at least about 5%, at least about 10%, at least about 20%, at least about 30% or more fewer regulatory T-cells than a healthy individual when comparing the numbers of T-cells in the same volume of blood. In still other aspects, the patient suffers from an autoimmune disease due to the presence of responder T-cells that are resistant to suppression by regulatory T-cells. In yet other aspects, the patient suffers from an autoimmune disease due to the presence of higher numbers of responder T-cells than in a healthy individual. In these aspects, the patient has at least about 5%, at least about 10%, at least about 20%, or at least about 30% or more responder T-cells than a healthy individual when comparing numbers of responder T-cells in the same volume of blood. In some aspects, the patient suffers from an autoimmune disease as a result of a combination of factors. *See e.g*., Costantino et al. (2008) Eur. J. Immunol. 38(4):921-924; Baecher-Allan et al. (2004) Seminars in Immunol. 16:89-97.

The term "autoimmune disease" as used herein is any disease that arises from an inappropriate immune response of a patient's body against substances and tissues normally present in the body. In an embodiment as defined in the claims, the autoimmune disease is selected from acute disseminated encephalomyelitis, Addison's disease, agammaglobulinemia, age-related macular degeneration, alopecia areata, amyotrophic lateral sclerosis, ankylosing spondylitis, antiphospholipid syndrome, antisynthetase syndrome, atopic allergy, atopic dermatitis, autoimmune aplastic anemia, autoimmune cardiomyopathy, autoimmune enteropathy, autoimmune hemolytic anemia, autoimmune hepatitis, autoimmune inner ear disease, autoimmune lymphoproliferative syndrome, autoimmune peripheral neuropathy, autoimmune pancreatitis, autoimmune polyendocrine syndrome, autoimmune progesterone dermatitis, autoimmune thrombocytopenic purpura, autoimmune uticaria, autoimmune uveitis, Balo disease/Balo concentric sclerosis, Behçet's disease, Berger's disease, Bickerstaff s encephalitis, Blau syndrome, Bullous pemphigoid, cancer, Castleman's disease, celiac disease, Chagas disease, chronic inflammatory demyelinating polyneuropathy, chronic recurrent multifocal osteomyelitis, chronic obstructive pulmonary disease, Churg-Strauss syndrome, cicatricial pemphigoid, Cogan syndrome, cold agglutinin disease, complement component 2 deficiency, contact dermatitis, cranial arteritis, CREST syndrome, Crohn's disease, Cushing's syndrome, cutaneous leukocytoclastic angiitis, Dego's disease, Dercum's disease, dermatitis herpetiformis, dermatomyositis, diabetes mellitus type 1, diffuse cutaneous systemic sclerosis, Dressler's syndrome, drug-induced lupus, discoid lupus erythematosus, eczema, endometriosis, enthesitis-related arthritis, eosinophilic fasciitis, eosinophilic gastroenteritis, epidermolysis bullosa acquisita, erythema nodosum, erythroblastosis fetalis, essential mixed cryoglobulinemia, Evan's syndrome, fibrodysplasia ossificans progressive, fibrosing alveolitis, gastritis, gastrointestinal pemphigoid, glomerulonephritis, Goodpasture's syndrome, Graves' disease, Guillan-Barré syndrome, Hashimoto's encephalopathy, Hashimoto's thyroiditis, Henoch-Schonlein purpura, gestational pemphigoid, hidradenitis suppurativa, Hughes-Stovin syndrome, hypogammaglobulinemia, idiopathic inflammatory demyelinating diseases, idiopathic pulmonary fibrosis, idiopathic thrombocytopenic purpura, IgA nephropathy, inclusion body myositis, chronic inflammatory demyelinating polyneuropathy, interstitial cystitis, juvenile idiopathic arthritis, Kawasaki's disease, Lambert-Eaton myasthenic syndrome, leukocytoclastic vasculitis, lichen planus, lichen sclerosus, linear IgA disease, lupus erythematosus, Majeed syndrome, Ménière's disease, microscopic polyangiitis, mixed connective tissue disease, morphea, Mucha-Habermann disease, multiple sclerosis, myasthenia gravis, myositis, narcolepsy, neuromyelitis optica, neuromyotonia, occular cicatricial pemphigoid, opsoclonus myoclonus syndrome, Ord's thyroiditis, palindromic rheumatism, pediatric autoimmune neuropsychiatric disorders associated with streptococcus, paraneoplastic cerebellar degeneration, paroxysmal nocturnal hemoglobinuria, Parry Romberg syndrome, Parsonage-Turner syndrome, Pars planitis, pemphigus vulgaris, pernicious anaemia, perivenous encephalomyelitis, POEMS syndrome, polyarteritis nodosa, polymyalgia rheumatic, polymyositis, primary biliary cirrhosis, primary sclerosing cholangitis, progressive inflammatory neuropathy,psoriasis, psoriatic arthritis, pyoderma gangrenosum, pure red cell aplasia, Rasmussen's encephalitis, Raynaud phenomenon, relapsing polychondritis, Reiter's syndrome, restless leg syndrome, retroperitoneal fibrosis, rheumatoid arthritis, rheumatic fever, sarcoidosis, schizophrenia, Schmidt syndrome, Schnitzler syndrome, scleritis, scleroderma, serum sickness, Sjogren's syndrome, spondyloarthropathy, stiff person syndrome, subacute bacterial endocarditis, Susac's syndrome, Sweet's syndrome, sympathetic ophthalmia, Takayasu's arteritis, temporal arteritis, thrombocytopenia, Tolosa-Hunt syndrome, transverse myelitis, ulcerative colitis, undifferentiated connective tissue disease, urticarial vasculitis, vasculitis, vitiligo and Wegener's granulomatosis.

In a particular embodiment as defined in the claims, the autoimmune disease is selected from acute disseminated encephalomyelitis, age-related macular degeneration, alopecia areata, ankylosing spondylitis, antiphospholipid syndrome, autoimmune cardiomyopathy, autoimmune hemolytic anemia, autoimmune hepatitis, autoimmune inner ear disease, autoimmune lymphoproliferative syndrome, autoimmune peripheral neuropathy, autoimmune pancreatitis, autoimmune polyendocrine syndrome, autoimmune progesterone dermatitis, autoimmune thrombocytopenic purpura, autoimmune uticaria, autoimmune uveitis, Behçet's disease, celiac disease, Chagas disease, chronic obstructive pulmonary disease, cold agglutinin disease, Crohn's disease, Dercum's disease, dermatomyositis, diabetes mellitus type 1, endometriosis, eosinophilic gastroenteritis, gastrointestinal pemphigoid, glomerulonephritis, Goodpasture's syndrome, Graves' disease, Guillan-Barré syndrome, Hashimoto's encephalopathy, Hasimoto's thyroiditis, hidradenitis suppurativa, idiopathic thrombocytopenic purpura, interstitial cystitis, Kawasaki's disease, lupus erythematosus, mixed connective tissues disease, morphea, multiple sclerosis, myasthenia gravis, narcolepsy, neuromyotonia, opsoclonus myoclonus syndrome, pediatric autoimmune neuropsychiatric disorders associated with streptococcus, paroxysmal nocturnal hemoglobinuria, pemphigus vulgaris, pernicious anaemia, polymyositis, primary biliary cirrhosis, progressive inflammatory neuropathy, psoriasis, psoriatic arthritis, Renaud phenomenon, relapsing polychondritis, restless leg syndrome, rheumatoid arthritis, rheumatic fever, sarcoidosis, schizophrenia, scleroderma, Sjogren's syndrome, stiff person syndrome, temporal arteritis, transverse myelitis, ulcerative colitis, undifferentiated connective tissue disease, vasculitis, vitiligo, and Wegener's granulomatosis.

In an aspect, the autoimmune disease is an autoimmune disease of the eye. In an aspect, the autoimmune disease is selected from uveitis and age-related macular degeneration.

### 6.1. Methods of identifying a compound comprising an epitope that induces immune tolerance

As used herein, the term "compound comprising an epitope" includes a compound comprising a contiguous region of monomers that elicits immune tolerance in a patient suffering from an autoimmune disease. In certain embodiments as defined in the claims, the compound comprises an epitope (*i.e.,* the epitope is a subset of contiguous monomers of the compound), or the compound consists of the epitope (*i.e.,* the entire compound is the epitope). In various aspects, the epitope is a self-epitope of the patient. In other aspects, the epitope is a non-self epitope. In certain embodiments as defined in the claims, the epitope is organ specific, or the epitope is not organ specific. In various aspects, the epitope is a human epitope. In other aspects, the epitope is a non-human mammalian epitope. In still other aspects, the epitope is a bacterial epitope or a viral epitope. In some aspects, the epitope is a mixture of epitopes from different organisms.

It will be understood by the skilled artisan that, in addition to the epitope, in various aspects, the compound includes one or more types of monomers, including naturally-occurring amino acids, non-naturally occurring amino acids, nucleotides, and the like. In certain aspects, the epitope consists of amino acids, which can be naturally occurring or non-naturally occurring. In particular aspects, the epitope consists of at least 3, such as at least 4, such as at least 5 or such as at least 6 or more amino acids. In certain aspects, the compound can be a single compound or an aggregate of compounds (e.g., cross-linked compounds). In various aspects, the compound can be unmodified or can be directly or indirectly (*i.e.,* through a linking moiety) linked to another moiety, *e.g.,* a sugar, a fat, a label (*e.g.,* a fluorescent or radioactive label) or an additional therapeutic agent.

In various aspects, the compound comprising an epitope that induces immune tolerance in a patient is identified from a library or collection of compounds. In some aspects, the library is a library of biological epitopes. Accordingly, in certain embodiments as defined in the claims, the library is a library of organ specific epitopes. In these embodiments, the epitopes are restricted to a particular organ of the body, *e.g.,* the eye. Thus, in a particular embodiment, the library of organ specific epitopes is a library of S-antigen epitopes. In other embodiments as defined in the claims, the library is a library of epitopes that are not organ specific, *e.g.,* that are found throughout the body. An example of this embodiment is a library of HLA epitopes, such as a library of variant HLA epitopes (e.g., a library of HLA-B27 epitopes). In some embodiments as defined in the claims, the library can be a library of epitopes from the patient (a library of self epitopes) or a library of epitopes that are not from the patient (a library of non-self epitopes).

In a particular embodiment as defined in the claims, the library is a library of synthetic peptides. In some aspects, peptides are synthesized with a given length and a predetermined overlapping sequence so that the library encompasses a particular protein. *See, e.g.,* Gershoni et al. (2007) BioDrugs 21 (3): 145-56. In other aspects, peptide libraries are created using mass spectrometry, such as by Solid Phase Epitope Recovery (SPHERE). *See* Lawendowski et al. (2002) J. Immunol. 169:2414-21. In certain aspects, a library for use in the methods described herein includes a phage display library, a bacterial or yeast display library, an mRNA display library, a ribosomal display library, a polysomal display library and a peptide matrix. *See e.g.,* U.S. Patent Publication No. 2013/0004513 (Osterroth et al.).

In other embodiments as defined in the claims, the compound comprising an epitope that induces immune tolerance in a patient is identified from a collection of compounds. In these embodiments, combinatorial epitope collections are utilized. Accordingly, in certain embodiments, the collection comprises all permutations of a compound having 4 monomers. In certain embodiments, the compound is a peptide and the collection comprises all permutations of a tetrameric peptide with all 20 amino acids at each position such that the collection includes 20⁴ peptide tetramers. In other embodiments, the compound is a peptide and the collection comprises all permutations of a pentameric peptide with all 20 amino acids at each position such that the collection includes 20⁵ peptide pentamers.

In various aspects, the *in vitro* methods for identifying a compound comprising an epitope that induces immune tolerance in a patient suffering from an autoimmune disease comprise measuring responses of responder T-cells and regulatory T-cells from the patient and measuring responses of responder T-cells and regulatory T-cells from a healthy individual and comparing the various responses from the T-cells of the patient with responses from the T-cells of a healthy individual. As used herein, a "healthy individual" is an individual who does not suffer from an autoimmune disease.

As used herein, a "responder T-cell" or "T-resp" refers to T-cells that mount an immune response to antigens, such as antigens presented on antigen presenting cells. Specifically, T-resp cells referred to herein are cells that mount an immune response to antigens, and in this context, to self-antigens. T-resp cells can be polyclonal or antigen-specific. T-resp cells include T-cells with certain phenotypes, including, CD8⁺ cells, CD4⁺ T-cells, naive CD4⁺CD25⁻ T-cells, NK cells, cytotoxic T lymphocytes (CTL), and mature dendritic cells (DC).

As used herein, a "regulatory T-cell" or "T-reg" refers to T-cells that suppress an immune response of T-resp cells. In certain embodiments, T-reg cells have an anergic phenotype, *i.e.,* they do not proliferate in response to T-cell receptor stimulation. T-reg cells include T cells with particular phenotypes, including CD4⁺CD25⁺ T-cells, CD4⁺Foxp3⁺ T-cells, CD4⁺CD25⁺Foxp3⁺ T-cells, IL-10 producing CD4⁺ Tr1 cells, TGF-β producing Th3 cells, CD8⁺ NKT cells, CD4⁻CD8⁻ T-cells, γδ T-cells, thymic nT-reg cells, periphery induced i-Treg cells, tolerogenic dendritic cells (DC), CD4⁺CD127^{lo/-}T-cells, CD4⁺CD127^{lo/-}CD25⁺ T-cells, and the CD45RA⁺ subset of CD4⁺CD127^{lo/-}CD25⁺ T-cells. In various aspects, T-reg cells are negative for CD127 and positive for CD39. In other aspects, T-reg cells are induced from CD4+CD25⁻ cells by stimulation with irradiated allogenic stimulator PMBCs. In some aspects, the T-reg cells inhibit polyclonal T-resp cells. In other aspects, T-reg cells inhibit antigen-specific T-resp cells.

The skilled artisan will recognize that new phenotypes of T-reg cells and T-resp cells may be discovered. Accordingly, the present disclosure encompasses not only T-reg and T-resp cells as described above, but also any T-cell having the characteristics of T-reg cells or T-resp cells.

Accordingly, in various aspects, the present disclosure relates to methods of identifying a compound comprising an epitope that induces immune tolerance in a human patient suffering from an autoimmune disease comprising the step of identifying *in vitro* a compound from a library or collection of compounds that (i) elicits a response (RespH) from a responder T-cell of a healthy individual, (ii) elicits a response (RespP) from a responder T-cell of the patient, (iii) elicits a response (RegH) from a regulatory T-cell of a healthy individual and (iv) elicits a response (RegP) from a regulatory T-cell of the patient, wherein the compound that induces a RespH/RespP<1, a RegH/RegP≥1 or a RespH/RespP<1 and a RegH/RegP≥1 is the compound that induces immune tolerance in the patient. In certain aspects, the compound induces a RespH/RespP≤1. In some aspects, the compound is identified by a RespP that is greater than the RespH. In other aspects, the compound is identified by a RegH that is greater than the RegP. In still other aspects, the compound is identified by a RespP that is greater than the Resp H and by a RegH that is greater than the RegP.

In another aspect, the disclosure relates to a method of identifying a compound comprising an epitope that induces immune tolerance in a patient suffering from an autoimmune disease comprising the step of (a) identifying *in vitro* a compound from a library or collection of compounds that (i) elicits a response (RespP₁) from a responder T-cell of the patient; (ii) elicits a response (RespH) from a responder T-cell of a healthy individual wherein RespP₁/RespH >1; and (iii) elicits a response (RespP₂) from a responder T-cell of the patient in the presence of a responder T-cell antigen and a regulatory T-cell, wherein RespP₂/RespP₁< 1, and wherein the compound that induces a RespP₁/RespH >1 and RespP₂/RespP₁< 1 is identified as the compound that induces an immune tolerance.

In some aspects, the compound is identified by a RespP₁ that is greater than the RespH and by a RespP₁ that is greater than the RespP₂.

In an embodiment as defined in the claims, the disclosure relates to a method of identifying a compound comprising an epitope from a synthetic peptide library or collection of peptide compounds that induces immune tolerance in a human patient suffering from an autoimmune disease, comprising (i) exposing a human CD4⁺CD25⁺ cell to a compound, (ii) measuring the proliferation (Reg₁) of human CD4⁺CD25⁺ cells in the presence of the compound, and (iii) measuring the proliferation (Reg₂) of the human CD4⁺CD25⁺ cells in the absence of the compound, wherein the compound that induces Reg₁/Reg₂>1 is identified as the compound that induces immune tolerance in the patient. In a particular embodiment, step (ii) is performed after the compound is removed. In certain aspects the compound that induces Reg₁/Reg₂≥1. In various aspects, the cell proliferation in the presence of the compound is greater than the cell proliferation in the absence of the compound.

In some aspects, the T-reg cells are induced, *e*.*g*., from naive cells, before step (i). In certain aspects of this method, the CD4⁺CD25⁺ cells are isolated before being exposed to a compound. In some aspects, cells are isolated using commercially available isolation kits, such as magnetic bead isolation using antibodies that specifically bind to CD4 and/or CD25 and/or other cell surface markers. In certain aspects, kits using positive or a combination of negative and positive selection are used. In various aspects, the identification of specific T-cell phenotypes is carried out using flow cytometry. In a particular aspect, identification and/or separation is accomplished by FACs. In various aspects, the CD4⁺CD25⁺ cells are from a healthy individual.

In yet another aspect, the present disclosure relates to a method of identifying a compound comprising an epitope that induces immune tolerance in a patient suffering from an autoimmune disease, comprising identifying *in vitro* a compound from a library or collection of compounds that (i) elicits a response (RespH) from a T-resp of a healthy individual, and (ii) elicits a response (RespP) from a T-resp of the patient, wherein the compound that induces a RespH/RespP<1 is identified as the compound that induces immune tolerance in the patient. In some aspects, the compound induces a RespH/RespP≤1.

In certain aspects, the response of the responder T-cell of the patient in the presence of the compound is greater than the response of the responder T-cell of the healthy individual in the presence of the compound.

In various aspects, the present disclosure relates to a method of identifying a compound comprising an epitope that induces immune tolerance in a human patient suffering from an autoimmune disease using a mixed-cell assay. As used herein, the term "mixed-cell assay" refers to an assay that includes both (i) responsive T-cells and (ii) regulatory T-cells. Thus, in certain aspects, the method of identifying a compound comprises a step of identifying *in vitro* a compound that elicits a response (Presp) from a responder T-cell of a patient in the presence of regulatory T-cells of the patient that is greater than the response (Preg) elicited from the regulatory T-cells of the patient in the assay. In certain aspects the Presp/Preg > 1. In other aspects, the method comprises a step of identifying *in vitro* a compound that elicits a response (Hreg) from regulatory T-cells of a healthy individual in the presence of responder T-cells of the patient that is greater than the response (Presp) elicited from the responder T-cells of the patient in the assay. In some aspects, Hreg/Presp>1. In still other aspects, the method comprises a step of identifying *in vitro* a compound that elicits a response (Hresp) from responder T-cells from a healthy individual in the presence of regulatory T-cells from the patient that is greater than the response (Preg) from the regulatory T-cells of the patient in the assay. In certain aspects, Hresp>Preg. In still other aspects, the method comprises a step of identifying *in vitro* a compound that elicits a response (Hresp) from responder T-cells from a healthy individual in the presence of regulatory T-cells from the healthy individual that is less than the response (Hreg) elicited from the regulatory T-cells of the healthy individual in the assay. In some aspects, Hresp<Hreg. It will be evident to the skilled artisan that more than one of the mixed-cell assays can be performed in order to identify a compound comprising an epitope that induces immune tolerance in a human patient suffering from an autoimmune disease.

As used herein, a "response" from at T-reg cell or a T-resp cell is an indication that a T-cell is upregulated. In various aspects, the response includes one or more of upregulation of cell-surface markers, such as activation markers, cytokine synthesis and/or secretion, and cell proliferation (expansion). A T-cell response can be measured by any method known in the art. In particular embodiments as defined in the claims, a T-cell response is measured by T-cell proliferation. In these embodiments, T-cell proliferation is measured by cell counting, *e.g.,* using flow cytometry, and in particular, fluorescence-activated cell sorting (FACs)) based on the T-cell markers. In other embodiments, T-cell proliferation can be measured by [³H]-thymidine uptake. *See, e.g.,* Wallace et al. (2008) Cytometry A 73(11):1019-34. In certain aspects, T-cell proliferation can be measured using cell tracking dyes to label T-resp cells and monitor decreases in fluorescence associated with cell division. In some aspects in which a mixed-cell assay is used, T-reg cells and T-resp cells can be independently labeled with two readily distinguishable dyes in order to discriminate each T-cell population in cocultures. *See* Brusko et al. (2007) Immunol. Investigations 36:607-628. *See, e.g.,* Venken et al. (2007) J. Immunol. Methods 322:1-11. In still other aspects, the activity of T-cells can be assayed by cytokine secretion, which can be detected, *e.g.,* by an ELIspot assay. In still other aspects, activated T-cells can be assayed by detection of intracellular cytokine production by intracytoplasmic cytokine staining. Other assay formats for measuring T-cell responses will be known to the skilled artisan. *See, e.g.,* LiPira et al. (2010) J. Biomedicine and Biotechnol. 1-12. *See, e.g.,* Kruisbeek et al. Current Protocols in Immunology 3.12.1-3.12.20 (John Wiley & Sons, Inc., 2004).

In various aspects, the *in vitro* assays described herein are carried out in the absence of antigen presenting cells. In other embodiments, the assays are performed in the presence of antigen presenting cells, such as murine antigen presenting cells or irradiated human PMBCs. In still other aspects, T-reg cells and/or T-resp cells are labeled, *e*.*g*., by radioisotopes or fluorescent dyes. In various aspects, the assays are performed in the presence of cytokines. Various types of *in vitro* T-cell assays for determining the activity of T-reg and T-resp cells will be known to the skilled artisan. *See, e.g.,* Collison and Vignali (2011) Methods Mol. Biol. 707:21-37.

The nature of T-reg cells in autoimmune diseases has been found to be variable. For example, Yeh et al., 2009 (Arch Opthamology 127; 407-413) found that there was a significant difference in numbers of T-reg cells between uveitis patients with active disease (4.3%) and uveitis patients with inactive disease (6.2%). Ursaciuc et al. 2010 (Romanian Arch Microbiol Immunol 69; 79-84) found a reduced presence of T-reg cells in systemic autoimmune diseases (SAID) compared to rheumatoid arthritis (RA) and controls and even concluded that T-reg percentage was the only cellular criterion of SAID evaluation. On the other hand, increased numbers of T-reg cells have also been found in autoimmune diseases such as juvenile arthritis (Cao et al., 2003 Eur J Immunol 33; 215-233). Lastly, there are reports that the defect lies not in the number of T-reg cells but in a disruption of their suppressive capability found in studies of multiple sclerosis (Viglietta et al., 2004 J Exp. Med. 199; 971-979), psoriasis (Sugiyama et al., 2005 J.Immunol. 174; 164-173) and myasthenia gravis (Baladina et al., 2003 AmN Y Acad Sci 998; 275-277). As such, certain aspects described herein measure the ability of a compound to be used in a suppressive assay with T-reg cells derived from diseased and healthy donors, where the number of total T-cells needed to provide a sufficient level of T-reg derived suppression of T-resp cells activity can be compared for a fixed level of inhibition. Thus, an antigen linked to a defect in either numbers or quality of T-reg cells in patients with autoimmune conditions will be recognized by these means since a ratio of the total number of T-cells from diseased and normal donors should be the same if the number and quality of T-reg cells are the same in both sources, whereas the number of T-cells used to achieve the fixed level will be greater to compensate for a loss of suppressive capability due to either a defect in the number or quality of T-reg cells in a patient sample. Accordingly, in various aspects, the measured response of T-cells is normalized. In some aspects in which the patient has fewer T-reg cells than a healthy individual in the same volume of blood, the responder and/or regulatory T-cell response is normalized by the steps of (i) determining the total number of T-cells (all types) ("P1") from a healthy donor that provides an amount of T-reg cells that induces 50% suppression of the T-resp response; (ii) determining the total number of T-cells (all types) ("P2") from the donor suffering from an autoimmune disease that provides an amount of T-reg cells that induces suppression of 50% of the T-resp response; and (iii) calculating P1/P2 to determine the amount of T-reg cells that are lacking in the donor suffering from an autoimmune disease. A ratio of P1/P2 that is greater than 1 is an indication that the compound may have therapeutic value as a tolerogenic agent or an agent for inducing or expanding T-reg cells that recognize the compound.

Thus, in some aspects, the disclosure relates to a method of identifying a compound comprising an epitope that induces immune tolerance in a human patient suffering from an autoimmune disease comprising the steps of (a) identifying *in vitro* a compound from a library or collection of compounds that (i) elicits a response (RespH) from responder T-cells of a healthy individual; and (ii) elicits a response (RespP) from responder T-cells of the patient; (b) determining the total number of T-cells (PI) from the healthy individual that provides an amount of T-reg cells that induces 50% suppression of T-resp activity in the presence of said compound; and (c) determining the total number of T-cells (P2) from the patient that provides an amount of T-reg cells that induces 50% suppression of said T-resp activity in the presence of said compound, wherein the compound that induces a RespH/RespP<1, a P1/P2>1 or RespH/RespP<1 and a P1/P2>1 is identified as the compound that induces immune tolerance in the patient.

In other aspects, the screening methods identify an epitope from a library of biological epitopes for treating age-related macular degeneration. In various aspects, the compound is a peptide. In one aspect, the peptide has the sequence N-GEPIPVTVDVTNNTEKTVKK-C, the P-23 fragment of S-antigen ("P-23").

In some aspects, an assay described herein is performed in the presence of one or more additional agents. In certain embodiments as defined in the claims, an assay described herein is performed in the presence of an immune tolerance enhancer. As used herein, an "enhancer" is any compound or mixture of compounds that potentiates the immune suppressive response of T-reg cells. In certain aspects, the enhancer is required for T-reg cell expansion. In some embodiments as defined in the claims, the enhancer is used in the *in vitro* methods described herein. In other aspects, the enhancer is used in *in vivo* methods described herein. In still other aspects, the enhancer is used in both *in vitro* assays and *in vivo* methods. In some embodiments as defined in the claims, the enhancer is high molecular weight hyaluronic acid. As used herein, the term "high molecular weight hyaluronic acid" refers to hyaluronic acid having a molecular weight of at least about 1 x 10⁶ Da, such as of at least about 2 x 10⁶ Da, at least about 3 x 10⁶ Da, at least about 4 x 10⁶ Da, or more. *See e.g.,* Bollyky et al. (2007) J. Immunol. 179:744-747. Other enhancers include IL-2, IL-15, TGF-β, all-trans retinoic acid, rapamycin, anti-CD3, anti-CD28, vitamin D3, dexamethasone, IL-10, idolamine-2,3-dioxygenase, FTY720, a sphingosine kinase 1 inhibitor, cholera toxin B subunit, ovalbumin, Flt2L, sirolimus and anti-thymocyte globulin, CTLA-4/Ig, and mixtures thereof. *See, e.g.,* Viney et al. (1998) J. Immunol. 160(12):5815-25; Horwitz et al. (2004) Seminars in Immunol. 16:135-143; Daniel et al. (2007) J. Immunol. 178(2): 458-68; Weiner et al. (2011) Immunol Rev. 241(1):241-259; Ma et al. (2011) Int. Immunopharmacol. 11(5):618-29; Adriouch et al. (2011) Front. Microbiol. 2:199; Dons et al. (2012) Human Immunol. 73:328-334. In certain embodiments as defined in the claims, the enhancer is a sphingosine kinase 1 inhibitor as disclosed in U.S. Patent No. 8,872,888. It will be understood by the skilled artisan that newly discovered enhancers are contemplated for use in the instant disclosure.

In some embodiments as defined in the claims, the compound identified by a method described above is used as a reference sequence to search a library for additional compounds, which have homology to the reference sequence. In certain aspects, the reference sequence is the entire protein target sequence. In various aspects, the reference sequence and identified compounds are compared using a comparison window, a contiguous specific segment of the polypeptide sequence, which can have gaps compared to the reference sequence, for optimal alignment of peptides. In certain aspects, the comparison sequence is at least about 10 amino acids, at least about 15 amino acids, at least about 20 amino acids, or at least about 25 or more amino acids. Tools for aligning sequences for comparison are well known in the art and include CLUSTAL in the PC/Gene program (available from Intelligenetics, Mountain View, Calif.); the ALIGN program (Version 2.0) and GAP, BESTFIT, BLAST, FASTA, and TFASTA in the GCG Wisconsin Genetics Software Package, Version 10 (available from Accelrys Inc., 9685 Scranton Road, San Diego, Calif., USA). In certain aspects, compounds are chosen that at least about 30%, such as at least about 35%, at least about 40%, at least about 45%, at least about 50%, at least about 55%, at least about 60%, at least about 65%, at least about 70%, at least about 75%, at least about 80%, at least about 85%, or at least about 90% or more homology or identity to the reference sequence. The identified compound can then be assayed by a method described herein.

Accordingly, in some aspects, the present disclosure relates to methods of identifying a compound comprising an epitope that induces immune tolerance in a human patient suffering from an autoimmune disease comprising the steps of: (a) identifying *in vitro* a compound from a library or collection of compounds that (i) elicits a response (RespH) from a responder T-cell of a healthy individual, (ii) elicits a response (RespP) from a responder T-cell of the patient, (iii) elicits a response (RegH) from a regulatory T-cell of a healthy individual and (iv) elicits a response (RegP) from a regulatory T-cell of the patient, wherein the compound that induces a RespH/RespP<1, a RegH/RegP≥1 or a RespH/RespP<1 and a RegH/RegP≥1 is the compound that induces immune tolerance in the patient; (b) using the compound identified in step (a) as a reference sequence to search a library or collection of compounds for one or more compounds having homology to the reference sequence; (c) identifying one or more compounds having homology to the reference sequence; and (d) repeating step (a) with the one or more compounds identified in step (c).

In another aspect, the disclosure relates to a method of identifying a compound comprising an epitope that induces immune tolerance in a patient suffering from an autoimmune disease comprising the steps of (a) identifying *in vitro* a compound from a library or collection of compounds that (i) elicits a response (RespP₁) from a responder T-cell of the patient; (ii) elicits a response (RespH) from a responder T-cell of a healthy individual wherein RespP₁/RespH>1; and (iii) elicits a response (RespP₂) from a responder T-cell of the patient in the presence of a responder T-cell antigen and a regulatory T-cell, wherein RespP₂/RespP₁< 1, and wherein the compound that induces a RespP₁/RespH >1 and RespP₂/RespP₁< 1 is identified as the compound that induces an immune tolerance; (b) using the compound identified in step (a) as a reference sequence to search a library or collection of compounds for one or more compounds having homology to the reference sequence; (c) identifying one or more compounds having homology to the reference sequence; and (d) repeating step (a) with the one or more compounds identified in step (c).

In yet another aspect, the disclosure relates to a method of identifying a compound comprising an epitope from a library or collection of compounds that induces immune tolerance in a human patient suffering from an autoimmune disease, comprising (i) exposing a human CD4⁺CD25⁺ cell to a compound, (ii) measuring the proliferation (Reg₁) of human CD4⁺CD25⁺ cells in the presence of the compound, (iii) measuring the proliferation (Reg₂) of the human CD4⁺CD25⁺ cells in the absence of the compound, wherein the compound that induces Reg₁/Reg₂>1 is identified as the compound that induces immune tolerance in the patient; (iv) using the compound identified in step (iii) as a reference sequence to search a library or collection of compounds for one or more compounds having homology to the reference sequence; (v) identifying one or more compound having homology to the reference sequence; and (vi) repeating steps (i)-(iii) with one or more compounds identified in step (v).

In another aspect, the present disclosure relates to a method of identifying a compound comprising an epitope that induces immune tolerance in a patient suffering from an autoimmune disease, comprising (a) identifying *in vitro* a compound from a library or collection of compounds that (i) elicits a response (RespH) from a T-resp of a healthy individual, and (ii) elicits a response (RespP) from a T-resp of the patient, wherein the compound that induces a RespH/RespP<1 is identified as the compound that induces immune tolerance in the patient; (b) using the compound identified in step (a)(ii) as a reference sequence to search a library or collection of compounds for one or more compounds having homology to the reference sequence; (c) identifying one or more compounds having homology to the reference sequence; and (d) repeating step (a) with one or more compounds identified in step (c).

In certain aspects, the compound that is used as a reference sequence to search a library or collection of compounds is identified using a mixed-cell assay, as described above.

In various aspects, the present disclosure relates to a method of identifying a compound comprising an epitope that induces immune tolerance in a patient suffering from an autoimmune disease comprising the steps of (a) identifying *in vitro* a compound from a library or collection of compounds that (i) elicits a response (RespH) from responder T-cells of a healthy individual; and (ii) elicits a response (RespP) from responder T-cells of the patient; (b) determining the total number of T-cells (PI) from the healthy individual that provides an amount of T-reg cells that induces 50% suppression of T-resp activity in the presence of said compound; (c) determining the total number of T-cells (P2) from the patient that provides an amount of T-reg cells that induces 50% suppression of said T-resp activity in the presence of said compound, (d) identifying the compound that induces a RespH/RespP<1, a P1/P2>1 or RespH/RespP<1 and a P1/P2>1 as the compound that induces immune tolerance in the patient; (e) using the compound identified in step (d) as a reference sequence to search a library or collection of compounds for one or more compounds having homology to the reference sequence; (f) identifying one or more compounds having homology to the reference sequence; and (g) repeating steps (a)-(d) with one or more compounds identified in step (f).

In certain aspects, the one or more compounds identified in the foregoing methods as having homology to a reference sequence has at least about 60%, such as at least about 65%, at least about 70%, at least about 75%, at least about 80%, at least about 85%, at least about 90% or at least about 95% homology to the reference sequence. In other aspects, the one or more compounds identified as having homology to the reference sequence has more than about 95% homology to the reference sequence.

The skilled artisan will further appreciate that homology between a reference sequence (*i.e*.*,* a compound identified by an *in vitro* method described above in this section) and a compound identified as having homology to the reference sequence set forth in this section may indicate not only a tolerizing epitope candidate, but also, the etiology of the autoimmune disease in a particular subject. Accordingly, the present disclosure relates to a method of determining the etiology of an autoimmune disease in a patient comprising the steps of (i) identifying a compound comprising an epitope *in vitro* from a library or collection of compounds that induces immune tolerance in a human patient by any of the methods described herein; (ii) using the compound identified in step (i) as a reference sequence to search a library or collection of compounds for one or more compounds having homology to the reference sequence; (iii) identifying one or more compounds having at least about 60% homology to the reference sequence, wherein the etiology of the compound identified in step (iii) is indicative of the etiology of the autoimmune disease. In certain aspects, the method comprises a step (iii) of identifying one or more compounds having at least about 65%, at least about 70%, at least about 75%, at least about 80%, at least about 85%, at least about 90% or at least about 95% homology or more to the reference sequence.

It will be understood by the skilled artisan that a compound identified by the methods described herein may be optimized to improve efficacy by altering or augmenting certain properties. Accordingly, in some aspects, a compound may be optimized, *e.g.,* to improve solubility, absorption and/or stability of the compound, to prolong its half-life in the body, or to target a specific organ. In some aspects, optimization may include altering the molecular weight, length and chemical make-up of the compound. In one aspect, the compound is a peptide. In an aspect, a peptide can be optimized by, *e.g.,* adding or removing amino acids, introducing conservative or non-conservative amino acid substitutions at various positions, incorporating non-natural amino acids, and crosslinking to other peptides or non-peptide therapeutic agents.

The skilled artisan will appreciate that optimization of a compound identified by the methods set forth herein may be an iterative process, comprising alteration of the compound followed by retesting of the altered compound in an *in vitro* assay described herein.

The skilled artisan will further appreciate that the analyses set forth in this section could potentially lead to the design of more effective tolerizing epitopes and/or a therapeutic effector that could act directly on the disease condition.

In various aspects, a compound identified *in vitro* as described above in section 6.1 can be used as a reference sequence to search a library of compounds to identify one or more compounds that have homology to the reference sequence. In these aspects, the identified compound that has homology to the reference sequence is a compound that induces immune tolerance in a human patient suffering from an autoimmune disease, such as AMD or uveitis. In various aspects, the compound identified by this method is a protein or a peptide from a protein that belongs to the same class of proteins as the reference sequence (e.g., S-antigen). In other aspects, the compound identified by this method is a protein or a peptide from a protein that belongs to a class of proteins unrelated to the reference sequence (e.g., a bacterial protein). In various aspects, the reference sequence is P-23.

### 8. EXAMPLES

This section will describe the various different working examples that will be used to highlight the features of the disclosure. Examples which do not fall uder the subject-matter of the appended claims do not form part of the present invention.

### 8.1. Example 1: Immune cell reactivity to antigens in AMD patients

In our evaluation of patients with AMD, we have noted marked immunological similarities to patients with uveitis. In AMD patients, we have seen activation of the acquired immune system, evidence of antigen sensitization as measured by proliferative responses by T cells, elevation of IL-17 cytokines and other members of that family, upregulation of IL-17RC in the macula, and an M2 to M1 macular switch, all of which are seen in uveitis. These characteristics make AMD a promising candidate for down-regulatory immune therapy with oral administration of antigen. Accordingly, peripheral blood lymphocytes of AMD patients were tested to determine whether they would manifest the same type of response that blood cells from uveitis patients did in an earlier study. *See* deSmet et al. (2001) Investigative Ophthalmology &Visual Science 42(13):3233-38.

### Materials and Methods

Several 18-mer sequences were constructed from the sequence of the retinal S-antigen. The fragments were chosen based on the responses of blood cells from uveitis patients previously tested. *See* deSmet *et al.* 2001. Whole blood was collected from patients with dry AMD and healthy controls. All AMD patients had either small drusen, intermediate drusen or large drusen.

Mononuclear lymphocytes were separated on isolymph gradient (Gallard-Schlesinger, Carle Place, NY) from heparinized blood shortly after the sample was obtained. Cells were resuspended in RMPI 1640 with HEPES (Gibco, Grand Island, NY), supplemented with glutamine (2 mM), Penicillin (100 U/ml), streptomycin (100 µg/ml), and 10% commercial heat-inactivated human AB serum (Biocell Laboratories, Carson, CA). These cells were immediately placed in culture at a density of 2 x 10⁵ cells/well in the presence of antigen, in flat-bottomed, 96-well plates (Costar, Cambridge, MA). All assays were plated in triplicate. Antigen concentrations were either 20 or 100 µg/ml. Peptides were tested simultaneously. For control of immune reactivity, purified protein derivative (PPD; Parke-Davis, Morris Plains, NJ) and purified phytohemagglutinin (PHA; Murex Diagnostics, Dartford, UK) were also tested. For the last 12 hours before harvesting at day 5, each well was pulsed with [3H]thymidine (2Ci/mmol, 0.5 µCi per 10 µl/well; New England Nuclear, Boston, MA).

### Results

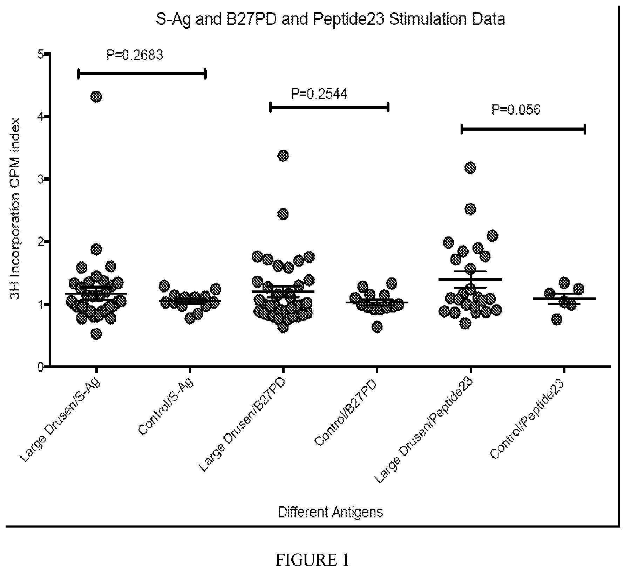

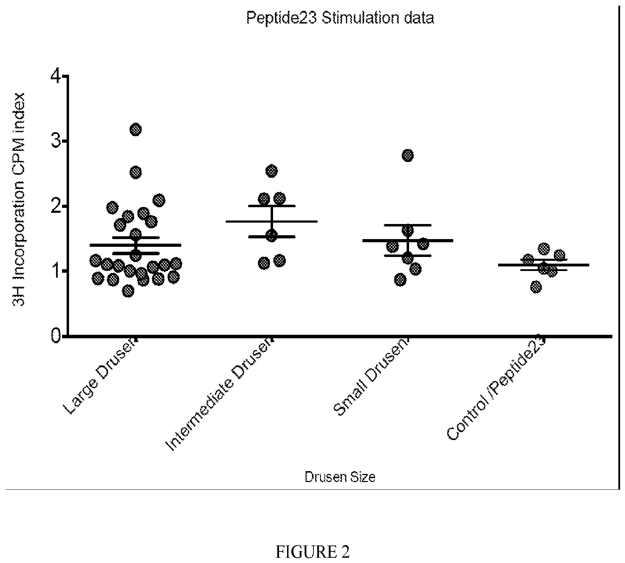

As shown in FIGS. 1 and 2, AMD patients' lymphocytes proliferated in the presence of whole S-antigen as well as to the BP27PD and Peptide 23 ("P-23") fragments. A large number of AMD patients responded best to P-23. In addition, FIG. 2 shows that lymphocytes from a significant number of AMD patients with small, intermediate and large drusen proliferated in the presence of P-23, and that lymphocytes from patients with large drusen had the best response.

### 8.2. Example 2: Peptide P-23 as AMD oral tolerizing agent

The objective of this study is to evaluate the safety and efficacy of the peptide P-23 as a long term method to prevent the development of more advanced AMD employing oral tolerance. Oral tolerance is investigated in patients with intermediate drusen who have a high risk of developing intermediate (large drusen with or without pigmentary changes) or late AMD. The primary outcome is the development of large drusen or late AMD. An important secondary outcome is defined as a mean change drusen volume on SD-OCT over 5 years without progression to geographic atrophy or neovascular disease. Participants who progress to advanced disease are considered treatment failures and censured from the drusen change analysis at the time late AMD develops.

Drusen is measured by the use of the scanning laser ophthalmoscopy (SLO). In a pilot study, drusen number and area grades were significantly higher using the right side (AR) and left side (AL) in which the laterally scattered light is captured (retromode). *See* Diniz et al. (2013) Br. J. Ophthalmol. 97(3):285-90. Use of the lateral confocal aperture may highlight subclinical drusen and aid in monitoring disease progression and response to emerging non-neovascular AMD therapies.

### Target Population

Participants have early and intermediate AMD with intermediate drusen in both eyes or large drusen (with or without pigment changes) in one eye and intermediate drusen in the fellow eye. All study eyes have intermediate drusen (<63 µm).

### Methods

This is a 5-year double-masked randomized clinical trial of 145 participants to assess the safety and efficacy of oral tolerance induction using drusen volume on OCT as a clinical end point. Patients are randomized 1:1 to P-23 fragment of retinal S-Antigen 4 mg oral daily or placebo oral daily.

### Study Outcome

The primary outcome is the development of large drusen or late AMD in patients with bilateral medium drusen or eyes whose fellow eye has large drusen. An important secondary outcome is defined as a mean change drusen volume on SD-OCT over 5 years without progression to geographic atrophy or neovascular disease. Participants who progress to advanced disease are considered treatment failures and censured from the drusen change analysis at the time late AMD develops.

Other secondary outcomes in study eyes include:
- Progression from intermediate drusen to large drusen or late AMD
- Change in Dark Adaptation time
- Mean change in best-corrected ETDRS (Early Treatment of Diabetic Retinopathy Study protocol) visual acuity from baseline to year-1.
- Changes in autofluorescence patterns on fundus autofluorescence photography
- Correlation with levels of serum inflammatory cytokines
- Correlation with flow cytometry evaluating T regulatory cells
- Correlation with epigenetic changes (demethylation of interleukin-17 receptor C)
- Changes in chromaticity coordinates on Cambridge color test (Regan et al., 1994)
- Safety outcomes
- Changes in drusen volume through year-5 (USC protocol)

### Sample Size Consideration

Detecting a 50% decrease in the development of large drusen or late AMD in patients with bilateral medium drusen or eyes whose fellow eye has large drusen, requires 132 patients with an α of 0.05 and β of 0.2. A 10% adjustment for loss to follow-up and non-compliance would increases the required sample size to 145.

Using a 0.041 mm change in cube root drusen volume, as compared to the reference mean change, has a power of 80.6%. This is based on a 0.16 mm mean change in cube root volume for this drusen size population (Yehoshua and Gregori, 2011) and reflects a mean change of -0.025 mm in cube root volume in the treatment group.

### Hazards and Discomforts

Possible complications associated with the study may include:
- Temporary gastrointestinal upset from either placebo and/or P-23.
- Transient ocular discomfort from ocular examination.
- Temporary discomfort, bruising or infection from blood draw.

All publications, patents, patent applications and other documents cited in this application are hereby incorporated by reference in their entireties for all purposes to the same extent as if each individual publication, patent, patent application or other document were individually indicated to be incorporated by reference for all purposes.

While various specific embodiments have been illustrated and described, it will be appreciated that various changes can be made without departing from the spirit and scope of the invention(s).

### 8.3. Example 3: Activation of T-reg cells and analysis of suppressive function

### Preparation of cells and solutions

CD4⁺CD25⁻ and CD4⁺CD25⁺ T-cell suspensions in RPMI-10 are prepared as described in Thornton (2003) Current Protocols in Immunology, Unit 3.5A (DOI: 10.1002/0471142735.im0305as57). Cells are counted and CD4⁺CD25⁻ and CD4⁺CD25⁺ cells are adjusted to 1 x 10⁶ cells/mL with RPMI-10 medium.

Accessory cells in RPMI-10 are prepared as described in Thornton (2003). Cells are counted and adjusted to 1 x 10⁶ cells/mL with RPMI-10. The following working solutions are prepared: 1 µg/mL anti-CD3 in RPMI-10; 200 U/mL IL-2 in RPMI-10; and 2 µg/mL anti-CD28 in RPMI-10.

50 µL of CD4⁺CD25⁻ cells are added to each of nine wells of a 96-well flat-bottom microtiter plate and 50 µL of CD4⁺CD25⁺ cells are added to each of nine wells of a 96-well flat-bottom microtiter plate. 50 µL of accessory cells and 50 µL of 1 50 µg/mL anti-CD3 are added to each of the wells. 50 µL of 200 U/mL IL-2 are added to three wells of the CD25⁻ cells and three wells of the CD25⁺ cells.

### Suppressive function assay

50 µL of CD4⁺CD25⁺ cells are added to three wells of a 96-well microtiter plate. A series of 3-4 two-fold dilutions of the CD4⁺CD25⁺ cells are made and control wells containing only 50 µL of RPMI-10 medium are included. After serial dilution, the starting number of cells in the wells are: 5 x 10⁴ CD25⁺ cells/well, 2.5 x 10⁴ CD25⁺ cells/well, 1.25 x 10⁴ CD25⁺ cells/well, 0.625 x 10⁴ CD25⁺ cells/well and 0.3 x 10⁴ CD25⁺ cells/well.

50 µL of CD4⁺CD25⁻ cells, 50 µL of accessory cells and 50 µL of 1 µg/mL anti-CD3 are added to the wells. The microtiter plates are placed in a 37°C, 5%-7% CO₂ humidified incubator for 3 days (about 66 hours).

On the morning of the third day [³H]thymidine is added to each well and plates are returned to the incubator to pulse for 6-8 hours. Cells are harvested using a semiautomated sample harvester and counts per minute are measured in a β scintillation counter.

### Results

CD4⁺CD25⁺ cells are non-responsive to stimulation with anti-CD3 and accessory cells. Addition of anti-CD28 to CD4⁺CD25⁺ cells stimulated with anti-CD3 and accessory cells does not restore proliferation of these cells. The addition of anti-CD28 to CD4+CD25-cells enhances their proliferation. Addition of IL-2 to CD4⁺CD25⁺ cells results in proliferation of these cells. Addition of CD4⁺CD25⁺ cells to CD4⁺CD25⁻ cells results in a dose-dependent decrease in the proliferation of CD4⁺CD25⁻ cells.

### 8.4. Example 4: Activation and expansion of CD4+CD25+ T-cells and analysis of suppressive function

### Activation of CD4⁺CD25⁺ T-cells

CD4⁺CD25⁺ T-cells are purified in complete RMPI-10 medium supplemented with 100 U/mL IL-2 as described in Thornton (2003) Current Protocols in Immunology, Unit 3.5A (DOI: 10.1002/0471142735.im0305as57). CD4⁺CD25⁺ cells are counted and adjusted to 1 x 10⁶ cells/mL with RPMI-10/IL-2.

A working solution of 5 µg/mL anti-CD3 in PBS is prepared. 300 µL of anti-CD3 solution is added to each well of a 24-well plate. Number of wells to be coated is based on anticipated yield of CD4⁺CD25⁺ cells. Plates are incubated for 90 min in a 37°C, 5%-7% CO₂ humidified incubator. Antibody is removed from the plates and wells are washed 2x with PBS to remove excess antibody. 1 mL containing 1 x 10⁶ CD4⁺CD25⁺ cells are added to the wells. Plates are placed in a 37°C, 5%-7% CO₂ humidified incubator for 3 days. CD4⁺CD25⁺ cells are fully activated but are not greatly expanded.

After three days, cells are split 1:3 or 1:4 in RPMI-10 medium supplemented with 100 U/mL IL-2 and are returned to the a 37°C, 5%-7% CO₂ humidified incubator.

### Suppressive function assay

Activated CD4⁺CD25⁺ cells are harvested by pipetting up and down rigorously. Cells are centrifuged for 10 min at 200xg (Sorvall H-1000B rotor at approx. 1000 rpm) at 4°C. Cells are washed 2x to completely remove remaining IL-2 and resuspend in RPMI-10. Cells are adjusted to 1 x 10⁶ cells/mL with RPMI-10.

CD4⁺ T-cell suspension in RPMI-10 is prepared from TCR transgenic mice as described in Unit 3.5A of Thornton (2003). CD4⁺ cells are counted and adjusted to 1 x 10⁶ cells/mL with RPMI-10. Antigen at 4x is diluted to the desired final concentration with RPMI-10. 50µL of CD4⁺CD25⁺ cells are added to three wells of a 96-well microtiter plate. A series of 3-4 two-fold dilutions of CD4⁺CD25⁺ cells are made and control wells containing 50 µL of RPMI-10 are included. After serial dilution, the starting number of cells in the wells are: 5 x 10⁴ CD25⁺ cells/well, 2.5 x 10⁴ CD25⁺ cells/well, 1.25 x 10⁴ CD25⁺ cells/well, 0.625 x 10⁴ CD25⁺ cells/well and 0.3 x 10⁴ CD25⁺ cells/well.

50 µL TCR Tg CD4+ cells, 50 µL of accessory cells and 50 µL of antigen are added to each well. The microtiter plates are placed in a 37°C, 5%-7% CO₂ humidified incubator for 3 days (about 66 hours).

On the morning of the third day [³H]thymidine is added to each well and plates are returned to the incubator to pulse for 6-8 hours. Cells are harvested using a semiautomated sample harvester and counts per minute are measured in a β scintillation counter.

### 8.5. Example 5: In vitro identification of a compound comprising an epitope that induces immune tolerance

Overlapping oligomeric peptide determinants of human HLA-B27 (Accession no. CAA27578.1) spanning the length of the protein are synthesized on an automated peptide synthesizer (Intavis, AG, Koeln, Germany). Each peptide is 15 amino acids in length and overlaps the previous peptide by 3 amino acids. Peptides are purified by HPLC to at least 95% purity. The amino acid composition of peptides is verified using amino acid analysis and automated gas-phase sequencing.

CD4⁺CD25⁺ cells are prepared as described in Example 3, above. 50 µL of CD4⁺CD25⁺ cells, 50 µL of accessory cells and 50 µL of 1 µg/mL anti-CD3 are added to the wells of a 96-well microtiter plate. HLA-B27 peptide is added to each well except for the control wells. All peptides are assayed in triplicate (3 wells each). The microtiter plates are placed in a 37°C, 5%-7% CO₂ humidified incubator for 3 days (about 66 hours).

On the morning of the third day [³H]thymidine is added to each well and plates are returned to the incubator to pulse for 6-8 hours. Cells are harvested using a semiautomated sample harvester and counts per minute are measured in a β scintillation counter.

The peptide that elicits the largest CD4⁺CD25⁺ cell proliferation as measured by levels of [³H]thymidine as compared CD4⁺CD25⁺ cell proliferation in the absence of peptide is the peptide that elicits immune tolerance in a patient.

### 8.6. Example 6: Administration of T-reg cells to patients suffering from type-I diabetes mellitus

T-reg cells from partially HLA-matched healthy individuals are prepared as set forth in Trzonkowski et al. (2009) Clin. Immunol. 133:22-26 and Marek et al. (2011) Cell Transplant 12:1747-1758. T-regs are cultured in the presence of 10% autologous serum, IL-2 (1000 U/mL) and clinical-grade anti-CD3/anti-CD28 beads in a 1:1 ratio with cells. Cells are cultured for about 10 days to 2 weeks, but no longer than 2 weeks.

T-reg cells for infusion are washed out completely, suspended in 250 mL 0.9% NaCl and transferred in slow infusion to a patient under anesthesia within 1 hour. T-regs are administered in a dose from 10 x 106/kg body weight to 20 x 10⁶ /kg body weight.

The endpoint is fasting C-peptide, HbA_{1c} level and insulin requirement. The percentage of T-regs in the patient's blood after 2 weeks, 2 months, 4 months and 6 months is assayed. If the percentage of T-regs drops by 50%, a compound as identified by the methods described herein is administered to the patient.

### 8.7. Example 7: Identification of peptides with sequence homology to P-23

The receptor of IL-17 has been shown to be hypomethylated and over-expressed in patients with age-related macular degeneration. IL-17RC, an allelic form of the IL-17 receptor, is highly expressed in macular tissue of AMD patients, but not in tissue of healthy individuals. *See* Wei et al. (2012) Cell Reports 2:1151-1158; and WO 2012/103187.

The P-23 peptide of S-antigen elicits a response from responder T-cells of AMD patients, but does not elicit a response from responder T-cells of healthy individuals. *See* Figure 1. A BLAST search using P-23 as the query sequence was performed and showed that there is significant homology between the P-23 peptide of S-antigen and IL-17RC. *See* Table 1. The homology between IL-17RC isomers 5 and 6 and P-23 is the result of a deletion of Exon 12 of the IL-17RC gene and fusion of Exons 11 and 13. *See* Haudenschild et al. (2002) J. Biol. Chem. 277(6):4309-4316, Figures 1 and 2. IL-17RC isoforms that have a deletion of Exon 12 cannot bind IL-17A or IL-17F. *See* Kuestner et al. (2007) J. Immunol. 179:5462-5473.

In addition, the BLAST search showed that P-23 has significant homology not only to IL-17RC isoforms, but also to a number of sequences from pathogenic organisms. *See* Table 1. This homology to pathogenic organisms in the case of AMD raises the question of whether infection by one or more infectious agents that have proteins that are homologous to proteins in the human body results in an aberrant immune response that leads to autoimmune disease.

Accordingly, epitopes that are demonstrated to react with regulatory T-cells and/or responder T-cells, and that belong to one class of protein, such as S-antigen, should be analyzed for homology with unrelated proteins from all sources, including humans and infectious agents. Such analyses could potentially lead to the etiology of an autoimmune disease, as well as to the design of more effective tolerizing epitopes and/or therapeutic agents. Furthermore, epitope homology may indicate not only a tolerizing epitope candidate, but also, a therapeutic effector that could directly act on the disease condition, *e*.*g*., that acts as an anti-inflammatory for a particular autoimmune disease.

**Table 1**

| **Results of P-23 (GEPIPVTVDVTNNTEKTVKK) BLAST Search** | | | |
|---|---|---|---|
| | **Alignment** | **Source of identified peptide** | **Identity** |
| Query Subject | VTVDVTNNTEK | Interleukin-17 receptor C isoform 6 precursor (Homo Sapiens) NP_ 001190194.1 | 73% |
| | VTVDV N EK | | |
| | VTVDV-NSSEK | | |
| Query Subject | VTVDVTNNTEK | Interleukin-17 receptor C isoform 5 precursor (Homo Sapiens) NP_001190193.1 | 73% |
| | VTVDV N EK | | |
| | VTVDV-NSSEK | | |
| Query Subject | GEPIPVTVDVTNNTEKTVKK | Dihydropicolinate from *Caenorhabditis elegans* | 60% |
| | GE I V V + NN KTVKK | | |
| | GESISVNVHIQNNSNKTVKK | | |
| Query Subject | GEPIPVTVDVTN | Probe for diagnosis of *Aspergillus fumigatus* | 83% |
| | GEPI VTV VTN | | |
| | GEPITVTVSVTN | | |
| Query Subject | GEPIPVTVDVTN | β-glucosidase from *Aspergillus fumigatus* | 83% |
| | GEPI VTV VTN | | |
| | GEPITVTVSVTN | | |
| Query Subject | IPVTVDVTNN | Probe for diagnosis of *Enterococcus faecalis* | 80% |
| | IPVT+DVT+N | | |
| | IPVTIDVTDN | | |

## Claims

1. A method of identifying a compound comprising an epitope from a synthetic peptide library or peptide collection that induces immune tolerance in a human patient suffering from an autoimmune disease, comprising
(a) exposing isolated human CD4⁺CD25⁺ cells to the compound,
(b) measuring the proliferation (Reg₁) of the human CD4⁺CD25⁺ cells in the presence of the compound,
(c) measuring the proliferation (Reg₂) of the human CD4⁺CD25⁺ cells in the absence of the compound, wherein the compound that induces Reg₁/Reg₂>1 is identified as the compound that induces immune tolerance in the patient;
(d) using the compound identified in step (c) as a reference sequence to search a library or collection of compounds for one or more compounds having homology to the reference sequence;
(e) identifying one or more compounds having homology to the reference sequence; and
(f) repeating steps (a)-(c) with one or more compounds identified in step (e).

2. The method of claim 1, wherein said cell proliferation is measured by incorporation of ³H or by monitoring decreases in fluorescence.

3. The method of claim 1, wherein the epitope is organ specific, or not organ specific.

4. The method of claim 1, wherein the library is a library of HLA epitopes.

5. The method of claim 1, wherein the library is a library of HLA-B27 epitopes, or of S-antigen epitopes.

6. The method of claim 1, wherein the collection includes all permutations of a pentameric or tetrameric peptide.

7. The method of claim 1, which is performed in the presence of an additional agent selected from 11-2, rapamycin, CD3 and CD28.

8. The method of claim 1, which is done in the presence of an enhancer of immune tolerance selected from the group consisting of high molecular weight hyaluronic acid, IL-2, IL-15, TGF-P, all-trans retinoic acid, rapamycin, anti-CD3, anti-CD28, vitamin D3, dexamethasone, IL-10, idolamine-2,3-dioxygenase, FTY720, a sphingosine kinase 1 inhibitor, cholera toxin B subunit, ovalbumin, sirolimus and anti-thymocyte globulin, CTLA-4/Ig, and mixtures thereof.

9. The method of claim 1, wherein the autoimmune disease is selected from the group consisting of acute disseminated encephalomyelitis, Addison's disease, agammaglobulinemia, age-related macular degeneration, alopecia areata, amyotrophic lateral sclerosis, ankylosing spondylitis, antiphospholipid syndrome, antisynthetase syndrome, atopic allergy, atopic dermatitis, autoimmune aplastic anemia, autoimmune cardiomyopathy, autoimmune enteropathy, autoimmune hemolytic anemia, autoimmune hepatitis, autoimmune inner ear disease, autoimmune lymphoproliferative syndrome, autoimmune peripheral neuropathy, autoimmune pancreatitis, autoimmune polyendocrine syndrome, autoimmune progesterone dermatitis, autoimmune thrombocytopenic purpura, autoimmune uticaria, autoimmune uveitis, Balo disease/Balo concentric sclerosis, Behçet's disease, Berger's disease, Bickerstaff's encephalitis, Blau syndrome, Bullous pemphigoid, cancer, Castleman's disease, celiac disease, Chagas disease, chronic inflammatory demyelinating polyneuropathy, chronic recurrent multifocal osteomyelitis, chronic obstructive pulmonary disease, Churg-Strauss syndrome, cicatricial pemphigoid, Cogan syndrome, cold agglutinin disease, complement component 2 deficiency, contact dermatitis, cranial arteritis, CREST syndrome, Crohn's disease, Cushing's syndrome, cutaneous leukocytoclastic angiitis, Dego's disease, Dercum's disease, dermatitis herpetiformis, dermatomyositis, diabetes mellitus type 1, diffuse cutaneous systemic sclerosis, Dressler's syndrome, drug-induced lupus, discoid lupus erythematosus, eczema, endometriosis, enthesitis-related arthritis, eosinophilic fasciitis, eosinophilic gastroenteritis, epidermolysis bullosa acquisita, erythema nodosum, erythroblastosis fetalis, essential mixed cryoglobulinemia, Evan's syndrome, fibrodysplasia ossificans progressive, fibrosing alveolitis, gastritis, gastrointestinal pemphigoid, glomerulonephritis, Goodpasture's syndrome, Graves' disease, Guillan-Barré syndrome, Hashimoto's encephalopathy, Hashimoto's thyroiditis, Henoch-Schonlein purpura, gestational pemphigoid, hidradenitis suppurativa, Hughes-Stovin syndrome, hypogammaglobulinemia, idiopathic inflammatory demyelinating diseases, idiopathic pulmonary fibrosis, idiopathic thrombocytopenic purpura, IgA nephropathy, inclusion body myositis, chronic inflammatory demyelinating polyneuropathy, interstitial cystitis, juvenile idiopathic arthritis, Kawasaki's disease, Lambert-Eaton myasthenic syndrome, leukocytoclastic vasculitis, lichen planus, lichen sclerosus, linear IgA disease, lupus erythematosus, Majeed syndrome, Meniere's disease, microscopic polyangiitis, mixed connective tissue disease, morphea, Mucha-Habermann disease, multiple sclerosis, myasthenia gravis, myositis, narcolepsy, neuromyelitis optica, neuromyotonia, occular cicatricial pemphigoid, opsoclonus myoclonus syndrome, Ord's thyroiditis, palindromic rheumatism, pediatric autoimmune neuropsychiatric disorders associated with streptococcus, paraneoplastic cerebellar degeneration, paroxysmal nocturnal hemoglobinuria, Parry Romberg syndrome, Parsonage-Turner syndrome, Pars planitis, pemphigus vulgaris, pernicious anaemia, perivenous encephalomyelitis, POEMS syndrome, polyarteritis nodosa, polymyalgia rheumatic, polymyositis, primary biliary cirrhosis, primary sclerosing cholangitis, progressive inflammatory neuropathy,psoriasis, psoriatic arthritis, pyoderma gangrenosum, pure red cell aplasia, Rasmussen's encephalitis, Raynaud phenomenon, relapsing polychondritis, Reiter's syndrome, restless leg syndrome, retroperitoneal fibrosis, rheumatoid arthritis, rheumatic fever, sarcoidosis, schizophrenia, Schmidt syndrome, Schnitzler syndrome, scleritis, scleroderma, serum sickness, Sjogren's syndrome, spondyloarthropathy, stiff person syndrome, subacute bacterial endocarditis, Susac's syndrome, Sweet's syndrome, sympathetic ophthalmia, Takayasu's arteritis, temporal arteritis, thrombocytopenia, Tolosa-Hunt syndrome, transverse myelitis, ulcerative colitis, undifferentiated connective tissue disease, urticarial vasculitis, vasculitis, vitiligo and Wegener's granulomatosis.

## Patentansprüche

1. Verfahren zum Identifizieren einer Verbindung, umfassend ein Epitop von einer synthetischen Peptidbibliothek oder Peptidsammlung, die bei einem menschlichen Patienten Immuntoleranz induziert, der an einer Autoimmunkrankheit leidet, umfassend:
(a) Aussetzen isolierter humaner CD4⁺CD25⁺-Zellen gegenüber der Verbindung,
(b) Messen der Proliferation (Reg₁) der humanen CD4⁺CD25⁺-Zellen in der Anwesenheit der Verbindung,
(c) Messen der Proliferation (Reg₂) der humanen CD4⁺CD25⁺-Zellen in der Abwesenheit der Verbindung, wobei die Verbindung, die die Reg₁/Reg₂>1 induziert, als die Verbindung identifiziert ist, die bei dem Patienten die Immuntoleranz induziert;
(d) Verwendung der in Schritt (c) identifizierten Verbindung als eine Referenzsequenz, um eine Bibliothek oder Sammlung von Verbindungen auf eine oder mehrere Verbindungen zu durchsuchen, die zu der Referenzsequenz eine Homologie aufweisen;
(e) Identifizieren einer oder mehrerer Verbindungen, die zu der Referenzsequenz eine Homologie aufweisen; und
(f) Wiederholen der Schritte (a) bis (c) mit einer oder mehreren in Schritt (e) identifizierten Verbindungen.

2. Verfahren nach Anspruch 1, wobei die Zellproliferation durch Einbau von ³H oder durch Kontrollieren der Abnahme von Fluoreszenz gemessen wird.

3. Verfahren nach Anspruch 1, wobei das Epitop organspezifisch oder nicht organspezifisch ist.

4. Verfahren nach Anspruch 1, wobei die Bibliothek eine Bibliothek von HLA-Epitopen ist.

5. Verfahren nach Anspruch 1, wobei die Bibliothek eine Bibliothek von HLA-B27-Epitopen oder von S-Antigen-Epitopen ist.

6. Verfahren nach Anspruch 1, wobei die Sammlung alle Permutationen eines pentameren oder tetrameren Peptids umfasst.

7. Verfahren nach Anspruch 1, das in der Anwesenheit eines zusätzlichen Agens, ausgewählt aus II-2, Rapamycin, CD3 und CD28, durchgeführt wird.

8. Verfahren nach Anspruch 1, das in der Anwesenheit eines Immuntoleranz-Verstärkers, ausgewählt aus der Gruppe bestehend aus Hyaluronsäure mit hohem Molekulargewicht, IL-2, IL-15, TGf-β, All-trans-Retinsäure, Rapamycin, anti-CD3, anti-CD28, Vitamin D3, Dexamethason, IL-10, ldolamin-2,3-dioxygenase, FTY720, einem Sphingosin-Kinase 1-Inhibitor, Cholera-Toxin B-Untereinheit, Ovalbumin, Sirolimus und anti-Thymocyt-Globulin, CTLA-4/lg und Gemischen daraus, durchgeführt wird.

9. Verfahren nach Anspruch 1, wobei die Autoimmunerkrankung ausgewählt ist aus der Gruppe bestehend aus akuter disseminierter Enzephalomyelitis, Morbus Addison, Agammaglobulinämie, altersbedingte Makuladegeneration, Alopecia areata, amyotrophe Lateralsklerose, Spondylitis ankylosans, Antiphospholipid-Syndrom, Antisynthetase-Syndrom, atopische Allergie, atopische Dermatitis, Autoimmun-Aplastische Anämie, Autoimmun-Kardiomyopathie, Autoimmunenteropathie, autoimmunhämolytische Anämie, Autoimmunhepatitis, Autoimmunerkrankung des Innenohrs, autoimmunes lymphoproliferatives Syndrom, autoimmune periphere Neuropathie, autoimmune Pankreatitis, polyendokrine Autoimmunerkrankung, autoimmune Progesteron-Dermatitis, Autoimmunthrombozytopenische Purpura, Autoimmunuticaria, Autoimmunuveitis, Balo-Krankheit / Balo-konzentrische Sklerose, Behget-Krankheit, Berger-Syndrom, Bickerstaff's Encephalitis; Blau-Syndrom, Bullöses Pemphigoid, Krebs, Castelman-Krankheit, Celiac-Krankheit, Chagas-Krankheit, chronische entzündliche demyelinierende Polyneuropathie, chronisch wiederkehrende multifokale Osteomyelitis, chronisch obstruktive Lungenerkrankung, Churg-Strauss-Syndrom, vernarbendes Pemphigoid, Cogan-Syndrom, kalte Agglutinin-Krankheit, Komplementkomponente 2-Mangel, Kontaktdermatitis, craniale Arteritis, CREST-Syndrom, Crohn-Krankheit, Cushing-Syndrom, kutane leukozytoclastische Angiitis, Dego-Krankheit, Dercum-Krankheit, Dermatitis herpetiformis, Dermatomyositis, Diabetes mellitus Typ 1, diffuser kutaner systemischer Sklerose, Dressler-Syndrom, drogeninduziertem Lupus, scheibenförmigem Lupus erythematodes, Ekzemen, Endometriose, Enthesitis-assoziierter Arthritis, eosinophiler Fasciitis, eosinophiler Gastroenteritis, Epidermolyse bullosa acquisita, Erythema nodosum, Erythroblastosis fetalis, essentieller gemischter Cryoglobulinämie, Evan-Syndrom, progressiver Fibrodysplasia ossificans, fibrosierender Alveolitis, Gastritis, gastrointestinalem Pemphigoid, Glomerulonephritis, Goodpasture-Syndrom, Graves-Krankheit, Guillan-Barre-Syndrom, Hashimoto-Enzephalopathie, Hashimoto-Thyroiditis, Henoch-Schönlein Purpura, Pemphigoid gestationis, Hidradenitis suppurativa, Hughes-Stovin-Syndrom, Hypogammaglobulinämie, idiopathischen entzündlichen demyelinierenden Erkrankungen, idiopathischer Lungenfibrose, idiopathischer thrombozytopenischer Purpura, IgA-Nephropathie, Einschlusskörpermyositis, chronisch entzündlicher demyelinisierender Polyneuropathie, interstitieller Blasenentzündung, juveniler idiopathischer Arthritis, Kawasaki-Krankheit, Lambert-Eaton-Myasthen-Syndrom, leukozytoklastischer Vaskulitis, Lichen planus, Lichen sclerosus, lineare IgA-Krankheit, Lupus erythematodes, Majeed-Syndrom, Meniere Krankheit, mikroskopischer Polyangiitis, gemischter Bindegewebserkrankung, Morphea, Mucha-Habermann-Krankheit, Multiple Sklerose, Myasthenia gravis, Myositis, Narkolepsie, Neuromyelitis optica, Neuromyotonie, okulärem vernarbendem Pemphigoid, Opsoclonus myoclonus-Syndrom, Ord-Thyreoiditis, palindromischem Rheuma, pädiatrischen autoimmunen neuropsychiatrischen Störungen im Zusammenhang mit Streptokokken, paraneoplastischer Kleinhirn-Degeneration, paroxysmaler nächtlicher Hämoglobinurie, Parry Romberg-Syndrom, Parsonage-Turner-Syndrom, Pars planitis, Pemphigus vulgaris, perniziöser Anämie, perivenöser Enzephalomyelitis, POEMS-Syndrom, Polyarteritis nodosa, rheumatischer Polymyalgie, Polymyositis, primärer biliärer Zirrhose, primärer sklerosierender Cholangitis, fortschreitende entzündliche Neuropathie, Psoriasis, Psoriasis-Arthritis, Pyoderma gangrenosum, reiner Aplasie der roten Blutkörperchen, Rasmussen-Enzephalitis, Raynaud-Phänomen, rezidivierender Polychondritis, Reiter-Syndrom, Restless-Leg-Syndrom, retroperitonealer Fibrose, rheumatoider Arthritis, rheumatischem Fieber, Sarkoidose, Schizophrenie, Schmidt-Syndrom, Schnitzler-Syndrom, Skleritis, Sklerodermie, Serumkrankheit, Sjögren-Syndrom, Spondyloarthropathie, Stiff-Person-Syndrom, subakuter bakterieller Endokarditis, Susac-Syndrom, Sweet-Syndrom, sympathischer Ophthalmie, Takayasu-Arteritis, temporale Arteritis, Thrombozytopenie, Tolosa-Hunt-Syndrom, transversaler Myelitis, Colitis ulcerosa, undifferenzierter Bindegewebserkrankung, Urtikaria-Vaskulitis, Vaskulitis, Vitiligo und Wegener-Granulomatose.

## Revendications

1. Méthode d'identification d'un composé comprenant un épitope à partir d'une bibliothèque de peptides synthétiques ou d'une collection de peptides qui induit une tolérance immunitaire chez un patient humain souffrant d'une maladie auto-immune, comprenant
(a) l'exposition de cellules CD4⁺CD25⁺ humaines isolées au composé,
(b) la mesure de la prolifération (Reg₁) des cellules CD4⁺CD25⁺ humaines en présence du composé,
(c) la mesure de la prolifération (Reg₂) des cellules CD4⁺CD25⁺ humaines en l'absence du composé, dans laquelle le composé qui induit Reg₁/Reg₂ > 1 est identifié comme le composé qui induit une tolérance immunitaire chez le patient;
(d) l'utilisation du composé identifié dans l'étape (c) en tant que séquence de référence pour la recherche dans une bibliothèque ou collection de composés d'un ou plusieurs composés ayant une homologie avec la séquence de référence;
(e) l'identification d'un ou plusieurs composés ayant une homologie avec la séquence de référence; et
(f) la répétition des étapes (a) à (c) avec un ou plusieurs composés identifiés dans l'étape (e).

2. Méthode selon la revendication 1, dans laquelle ladite prolifération cellulaire est mesurée par incorporation de ³H ou par surveillance de diminutions de fluorescence.

3. Méthode selon la revendication 1, dans laquelle l'épitope est spécifique d'un organe, ou non spécifique d'un organe.

4. Méthode selon la revendication 1, dans laquelle la bibliothèque est une bibliothèque d'épitopes de HLA.

5. Méthode selon la revendication 1, dans laquelle la bibliothèque est une bibliothèque d'épitopes de HLA-B27 ou d'épitopes d'antigène S.

6. Méthode selon la revendication 1, dans laquelle la collection englobe toutes les permutations d'un peptide pentamère ou tétramère.

7. Méthode selon la revendication 1, qui est mise en œuvre en présence d'un agent additionnel choisi parmi IL-2, la rapamycine, CD3 et CD28.

8. Méthode selon la revendication 1, qui est effectuée en présence d'un amplificateur de tolérance immunitaire choisi dans le groupe constitué par l'acide hyaluronique de masse moléculaire élevée, IL-2, IL-15, TGF-β, l'acide tout-trans-rétinoïque, la rapamycine, un anti-CD3, un anti-CD28, la vitamine D3, la dexaméthasone, IL-10, l'idolamine-2,3-dioxygénase, FTY720, un inhibiteur de sphingosine kinase 1, la sous-unité B de la toxine cholérique, l'ovalbumine, le sirolimus et la globuline anti-thymocytes, CTLA-4/Ig, et leurs mélanges.

9. Méthode selon la revendication 1, dans laquelle la maladie auto-immune est choisie dans le groupe constitué par l'encéphalomyélite aiguë post-infectieuse, la maladie d'Addison, l'agammaglobulinémie, la dégénérescence maculaire liée à l'âge, l'alopécie en aires, la sclérose latérale amyotrophique, la spondylarthrite ankylosante, le syndrome des antiphospholipides, le syndrome des antisynthétases, l'atopie, la dermite atopique, l'anémie aplasique auto-immune, la cardiomyopathie auto-immune, l'entéropathie auto-immune, l'anémie hémolytique auto-immune, l'hépatite auto-immune, une maladie de l'oreille interne auto-immune, le syndrome lymphoprolifératif auto-immun, la neuropathie périphérique auto-immune, la pancréatite auto-immune, le syndrome polyendocrinien auto-immun, la dermite de progestérone auto-immune, le purpura thrombopénique auto-immun, l'urticaire auto-immun, l'uvéite auto-immune, la maladie de Balo / sclérose concentrique de Balo, la maladie de Behcet, la maladie de Berger, l'encéphalite de Bickerstaff, le syndrome de Blau, la pemphigoïde bulleuse, un cancer, la maladie de Castleman, la maladie cœliaque, la maladie de Chagas, la polyneuropathie démyélinante inflammatoire chronique, l'ostéomyélite multifocale récurrente chronique, la broncho-pneumopathie chronique obstructive, le syndrome de Churg-Strauss, la pemphigoïde cicatricielle, le syndrome de Cogan, la maladie des agglutinines froides, l'insuffisance en composant 2 de complément, la dermatite de contact, l'artérite crânienne, le syndrome CREST, la maladie de Crohn, le syndrome de Cushing, l'angéite leucocytoclastique cutanée, la maladie de Dego, la maladie de Dercum, la dermite herpétiforme, la dermatomyosite, le diabète sucré de type 1, la sclérodermie généralisée cutanée diffuse, le syndrome de Dressler, le lupus d'origine médicamenteuse, le lupus érythémateux discoïde, l'eczéma, l'endométriose, l'arthrite liée à une enthésopathie, la fasciite à éosinophiles, la gastro-entérite à éosinophiles, l'épidermolyse bulleuse acquise, l'érythème noueux, l'érythroblastose du nouveau-né, la cryoglobuminémie mixte essentielle, le syndrome d'Evan, la fibrodysplasie ossifiante progressive, l'alvéolite fibrosante, la gastrite, la pemphigoïde gastro-intestinale, la glomérulonéphrite, le syndrome de Goodpasture, la maladie de Graves, le syndrome de Guillan-Barré, l'encéphalopathie de Hashimoto, la thyroïdite de Hashimoto, le purpura de Henoch-Schonlein, la pemphigoïde gestationnelle, l'hydrosadénite suppurée, le syndrome de Hughes-Stovin, l'hypogammaglobulinémie, les maladies démyélinantes inflammatoires idiopathiques, la fibrose pulmonaire idiopathique, le purpura thrombopénique idiopathique, la néphropathie à IgA, la myosite à corps d'inclusion, la polyneuropathie démyélinante inflammatoire chronique, la cystite interstitielle, l'arthrite juvénile idiopathique, la maladie de Kawasaki, le syndrome myasthénique de Lambert-Eaton, l'angéite leucocytoclastique, le lichen plan, le lichen scléreux, la maladie à IgA linéaire, le lupus érythémateux, le syndrome de Majeed, la maladie de Ménière, la polyangéite microscopique, la connectivité mixte, la morphée, la maladie de MuchaHabermann, la sclérose en plaques, la myasthénie grave, la myosite, la narcolepsie, la neuromyélite optique, la neuromyotonie, le pemphigoïde cicatricielle oculaire, le syndrome d'opsoclonie myoclonie, la thyroïdite d'Ord, le rhumatisme palindromique, les troubles neuropsychiatriques auto-immuns pédiatriques associés à Streptococcus, la dégénérescence cérébelleuse paranéoplasique, l'hémoglobinurie nocturne paroxystique, le syndrome de Parry Romberg, le syndrome de Parsonage-Turner, l'uvéite intermédiaire chronique idiopathique, le pemphigus vulgaire, l'anémie pernicieuse, l'encéphalomyélite périveineuse, le syndrome POEMS, la polyartérite noueuse, la polymyalgie rhumatismale, la polymyosite, la cirrhose biliaire primaire, la cholangite sclérosante primaire, la neuropathie inflammatoire progressive, le psoriasis, l'arthrite psoriasique, l'idiophagénédisme, l'érythroblastopénie pure, l'encéphalite de Rasmussen, le phénomène de Raynaud, la polychondrite récidivante, le syndrome de Reiter, le syndrome des jambes sans repos, la fibrose rétropéritonéale, la polyarthrite rhumatoïde, le rhumatisme articulaire aigu, la sarcoïdose, la schizophrénie, le syndrome de Schmidt, le syndrome de Schnitzler, la sclérite, la sclérodermie, la maladie sérique, le syndrome de Sjögren, la spondylarthropathie, le syndrome de l'homme raide, l'endocardite bactérienne subaiguë, le syndrome de Susac, le syndrome de Sweet, l'ophtalmie sympathique, l'artérite de Takayasu, l'artérite temporale, la thrombopénie, le syndrome de Tolosa-Hunt, la myélite transversale, la recto-colite hémorragique, la maladie du tissu conjonctif non différencié, l'angéite urticante, l'angéite, le vitiligo et la granulomatose de Wegener.
